# FASCICULE DE BREVET EUROPEEN

(11) **EP 0 704 210 B1**
(45) Date de publication et mention de la délivrance du brevet: **04.12.2002**
(21) Numéro de dépôt: 95402155.6
(22) Date de dépôt: 26.09.1995
(51) Int. Cl.: A61K 31/195, A61K 31/55, A61K 7/48, A61P 21/02

(54) **Utilisation d'une substance agoniste d'un récepteur associé à un canal chlore dans le traitement des rides**
Verwendung von einem Agonisten eines mit einem Chloridkanal assozierten Rezeptors zur Behandlung von Hautfalten
Use of an agonist of a receptor associated to a chloride channel in the treatment of wrinkles

(30) Priorité: 30.09.1994 FR 9411742
(43) Date de publication de la demande: 03.04.1996
(73) Titulaire: L'OREAL, 75008 Paris (FR)
(72) Inventeur: De Lacharriere, Olivier, F-75015 Paris (FR); Breton, Lionel, F-78000 Versailles (FR)
(74) Mandataire: Lhoste, Catherine

(56) Documents cités:
- GB-A- 2 142 237
- J. APPL. COSMETOL., vol. 12, no. 1, 1994, pages 1-9, XP002024589 P. MORGANTI: "Topical gelatin-glycine and alpha-hydroxy acids for photoaged skin."
- STN INTERNATIONAL, KARLSRUHE, XP002024590 & RO-B-85674,
- PATENT ABSTRACTS OF JAPAN vol. 17, no. 342 (C-1076) & JP 05 043448 A (KANEBO LTD.)
- PATENT ABSTRACTS OF JAPAN vol. 10, no. 31 (C-327) & JP 60 184005 A (KANEBO KK)
- STN INTERNATIONAL, KARLSRUHE, XP002024591 & JP-A-59053409,
- J. DERMATOL. SURG. ONCOL., vol. 18, no. 1, 1992, pages 17-21, XP002024592 J.D.A. CARRUTHERS: "Treatment of glabellar frown lines with C. botulinum-A exotoxin"

## Description

La présente invention se rapporte à l'utilisation d'une substance agoniste d'un récepteur associé à un canal chlore dans une composition cosmétique et/ou dermatologique, notamment en vue de traiter les rides et les ridules de la peau, ainsi qu'à la composition cosmétique et/ou dermatologique obtenue.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans rides, marques d'une peau jeune, d'autant plus que l'aspect physique induit sur le psychisme et/ou sur le moral. Or, il est important de se sentir physiquement et moralement jeune.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Mais, à ce jour, on ne sait pas agir sur les rides en intervenant sur les éléments musculaires présents dans la peau.

Il est connu que les muscles peauciers du visage sont sous le contrôle des afférences nerveuses motrices du nerf facial et que, par ailleurs, les cloisons interlobulaires de l'hypoderme contiennent en leur sein des fibres qui constituent un tissu musculaire strié (*panniculus carnosus*). D'autre part, il est également connu qu'une sous-population de fibroblastes du derme que l'on appelle myofibroblastes, présente des caractéristiques communes avec le tissu musculaire.

La demanderesse a notamment observé, dans certaines situations pathologiques et thérapeutiques, le rôle joué sur les rides du visage par les nerfs contrôlant l'ensemble de ce tissu musculaire. Ainsi, dans les atteintes du nerf facial, dans lesquelles la transmission de l'influx nerveux est interrompue et/ou amoindrie, on assiste dans le territoire d'innervation à une paralysie des muscles du visage. Cette paralysie faciale se traduit, entre autres signes cliniques, par une atténuation, voire une disparition des rides.

A l'inverse, dans les états d'hypercontraction musculaire de la face, la demanderesse a constaté une accentuation des rides du visage. De plus, elle a également observé une accentuation des rides du visage dans les états d'hypertonie musculaire de la maladie de Parkinson et des effets secondaires induits par les neuroleptiques.

Par ailleurs, il a été montré que la toxine botulique, utilisée à l'origine pour traiter les spasmes, pouvait agir sur les états de spasticité musculaire (voir A. Blitzer et al., Arch. Otolaryngol. Head Neck Surg., 1993, 119, pages 1018 à 1022) et sur les rides de la glabelle qui sont les rides inter sourcillières (voir J.D. Carruters et al., J. Dermatol. Surg. Oncol., 1992, 18, pages 17 à 21). En conséquence, il est possible d'agir par une action pharmacologique sur la composante nerveuse des rides. La toxine botulique agit directement au niveau de la jonction neuro-musculaire en bloquant l'action de l'acétylcholine sur la contraction musculaire.

La jonction entre un nerf et un muscle constitue la plaque neuro-musculaire, en amont de laquelle se trouve la voie nerveuse afférente appelée motoneurone. Par ailleurs, les membranes cellulaires de chaque fibre nerveuse comportent de nombreux canaux ioniques, et notamment des canaux chlore, aptes à laisser traverser l'élément correspondant sous forme ionique, et dans le cas des canaux chlore sous forme de chlorure. A ces canaux, sont associés des récepteurs neuronaux. Les récepteurs neuronaux associés aux canaux chlore sont notamment des récepteurs pour la glycine (les récepteurs glycine-strychnine sensibles) et des récepteurs pour le GABA (les récepteurs GABA_{A}).

Par ailleurs, on sait que dans le système nerveux central, il est possible de diminuer l'excitabilité du neurone par divers agents pharmacologiques agissant sur les récepteurs glycine-strychnine sensibles ou sur les récepteurs GABA_{A} du système nerveux central (voir W. Sieghart, Trends in Pharmacological Science, décembre 1992, vol. 131, pages 446 à 450). L'activation de ces récepteurs ouvre les canaux chlore et conduit à l'entrée d'ions chlorure, ce qui aboutit à une augmentation des ions chlorure dans les cellules de la fibre nerveuse et donc à une hyperpolarisation des neurones qui deviennent par voie de conséquence moins excitables. D'autre part, au niveau de la jonction neuro-musculaire, une diminution d'excitabilité du motoneurone entraîne une moindre stimulation de la fibre musculaire, provoquant ainsi son relâchement.

Après de nombreux tests cliniques, la demanderesse a pu déterminer que les fibres musculaires contractiles, qui se trouvent sous le contrôle direct de l'influx neuro-moteur, jouaient un rôle essentiel dans la pathogénie des rides et que la suppression de l'influx neuro-moteur atténuait non seulement les rides mais également les ridules et avait aussi un effet de "lissage" sur le microrelief cutané. Elle a aussi trouvé que le tissu cutané comportait des récepteurs associés aux canaux chlore, ce qui, jusqu'à présent, n'avait pas été envisagé. Elle a donc trouvé que l'on pouvait agir sur ces canaux pour relâcher ou relaxer ces tissus, et ainsi diminuer les rides et les ridules.

Personne n'avait, jusqu'à ce jour, établi un lien entre les canaux chlore des fibres nerveuses motrices du système nerveux périphérique cutané et les rides, et n'avait trouvé que l'on pouvait traiter les rides en agissant sur les canaux chlore par activation des récepteurs se trouvant dans ou au voisinage de ces canaux. Les substances qui peuvent activer les récepteurs des canaux chlore et donc entraîner l'entrée de chlorure dans les cellules, sont appelées substances agonistes.

Aussi, la présente invention se rapporte à l'utilisation telle que définie dans la revendication 1.

L'invention a également pour objet l'utilisation telle que définie dans la revendication 4.

« Intralésionnelle » ou « intraridaire » signifie que l'injection est effectuée dans ou sous le tissu cutané lésé, notamment dans la ride ou la ridule.

L'invention se rapporte encore à l'utilisation telle que définie dans la revendication 5.

L'invention se rapporte en outre à l'utilisation telle que définie dans la revendication 2.

La composition contenant l'agoniste selon l'invention peut être appliquée par voie topique ou par injection sous-cutanée et/ou intradermique « intralésionnelle » ou « intraridaire ».

Il existe plusieurs récepteurs associés au canal chlore. Il s'agit notamment des récepteurs glycine-strychnine sensibles et des récepteurs GABA_{A}, ces derniers comportant eux-mêmes plusieurs sous-unités constituées par le site GABA, le site benzodiazépine, un type de site stéroïde et le site aux barbituriques. Toutes les substances agissant comme agonistes de ces récepteurs ou sites peuvent être utilisées pour retâcher ou relaxer le tissu cutané conformément à l'invention.

Pour qu'une substance soit reconnue comme un agoniste des récepteurs des canaux chlore, elle doit répondre aux deux caractéristiques suivantes :
- pouvoir se fixer sélectivement sur au moins un des différents récepteurs associés au canal chlore ;
- montrer un effet de relaxation sur un tissu musculaire contracté.

La première caractéristique, qui consiste en la possibilité de se fixer sur un récepteur associé à un canal chlore, ne permet pas de distinguer une activité agoniste d'une activité antagoniste, mais elle permet de définir une affinité potentielle pour le récepteur.

La seconde caractéristique permet de sélectionner les agonistes. L'activité agoniste de la substance étudiée peut être mise en évidence par l'effet de relaxation qu'elle produit sur un tissu musculaire qui a été préalablement contracté par une substance antagoniste des canaux chlore. Comme substance antagoniste du canal chlore, on peut choisir les substances connues comme telles, et notamment les substances suivantes : bicuculline, strychnine, terbutyl-bicyclo-phosphorothionate et picrotoxine.

Comme substances agonistes utilisables dans l'invention pour activer les récepteurs à la glycine-strychnine sensibles, on peut citer , la sérine, la taurine, la β-alanine, ainsi que la N-(benzyloxycarbonyl)-glycine ou Z-glycine.

Comme substances agonistes utilisables dans l'invention pour activer les récepteurs au GABA_{A}, on peut citer, l'isoguvacine, l'acide isonipécotique, la 4,5,6,7-tétrahydroisoxazolo-(5,4-c)pyridin-3(2H)-one (THIP) ; les benzodiazépines telles que le nitrazépam (1,3-dihydro-7-nitro-5-phényl-2H-1,4-benzodiazépin-2-one), le diazépam (7-chloro-1,3-dihydro-1-méthyl-5-phényl-2H-1,4-benzodiazépin-2-one), le flunitrazépam (5-(2-fluorophényl)-1,3-dihydro-1-méthyl-7-nitro-2H-1,4-benzodiazépin-2-one), l'oxazépam (7-chloro-1,3-dihydro-3-hydroxy-5-phényl-2H-1,4-benzodiazépin-2-one) ; certains stéroïdes tels que l'alfaxalone (3-hydroxypregnane-11,20-dione); les barbituriques tels que le barbital (acide 5,5-diéthylbarbiturique), le pentobarbital (acide 5-éthyl-5-(1-méthylbutyl)barbiturique), le phénobarbital (acide 5-éthyl-5-phénylbarbiturique) et leurs sels.

Certes, il est connu d'utiliser le GABA et la glycine en association avec d'autres actifs pour lutter contre le vieillissement de la peau, mais personne jusqu'à ce jour n'avait trouvé leur utilisation dans une composition dermatologique injectable "intralésionnelle" ou "intraridaire" pour relaxer et relâcher le tissu cutané en vue de traiter les rides. Les actions généralement connues sont l'inhibition de l'élastase, l'effet sur le collagène et le renouvellement cellulaire.

En effet, il est connu dans l'état de la technique d'utiliser les aminoacides comme hydratants en vue d'améliorer l'état de la peau. En particulier, les associations d'aminoacides tels que la glycine, la taurine ou la β-alanine sous forme de mélanges peptidiques ont été utilisées dans des compositions cosmétiques destinées à traiter le vieillissement de la peau. Ainsi, le document FR-A-2546164 divulgue les propriétés d'inhibiteurs d'élastase des lipopeptides empêchant la dégradation des fibres d'élastine dans la peau, ce qui en fait des actifs antirides. Par ailleurs, le document US-A-5198465 divulgue que les aminoacides évitent les déficiences dans la synthèse du collagène, ce qui par conséquent permet d'éviter le vieillissement de la peau.

En outre, le document JP-A-05043448 divulgue que l'association de GABA et de diisopropylamine facilite le renouvellement de la peau et évite ainsi le vieillissement cutané.

Dans les compositions, l'agoniste d'un récepteur associé au canal chlore est utilisé de préférence en une quantité allant de 0,00001 à 20 % en poids par rapport au poids total de la composition, et en particulier en une quantité allant de 0,001 à 10 % en poids par rapport au poids total de la composition.

Les compositions peuvent se présenter sous toutes les formes galéniques normalement utilisées pour une application topique ou injectable « intralésionnelle » ou « intraridaire ».

Les quantités des différents constituants des compositions sont celles classiquement utilisées dans les domaines considérés et sont appropriées à leur forme galénique.

Pour une application topique, les compositions comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de traitement ou de soin cosmétique ou dermatologique pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou dermatologique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, les compositions cosmétiques ou dermatologiques peuvent contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou dermatologique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions, on peut notamment citer les actifs ayant un effet sur le traitement des rides ou des ridules, et en particulier les actifs kératolytiques. Par kératolytique, on entend un actif ayant des propriétés desquamantes, exfoliantes ou gommantes, ou un actif capable de ramollir la couche cornée.

Parmi ces actifs ayant un effet sur le traitement des rides ou des ridules que peuvent contenir les compositions, on peut en particulier citer les hydroxyacides et les rétinoïdes.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthylbenzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001 % à 5 % en poids par rapport au poids total de la composition.

Quand les compositions sont destinées à être injectées, elles peuvent se présenter sous forme de solutions contenant les excipients habituellement utilisés pour les injections, et par exemple sous forme d'une solution isotonique de chlorure de sodium.

Les exemples qui suivent sont donnés à titre illustratif afin de mieux faire comprendre l'invention. Les quantités indiquées sont des pourcentages en poids.

| **EXEMPLE 1 : Lotion de soin pour le visage** | |
|---|---|
| Z-Glycine | 8 % |
| Antioxydant | 0,05 % |
| Conservateur | 0,3 % |
| Ethanol (solvant) | 8 % |
| Eau | qsp 100 % |

La lotion obtenue agit sur les rides lors d'une utilisation répétée (application biquotidienne pendant un mois).

| **EXEMPLE 2 : Gel pour le soin du visage** | |
|---|---|
| Z-glycine | 5 % |
| Hydroxypropylcellulose (Ktucel H vendu par la société | |
| Hercules) (gélifiant) | 1 % |
| Conservateur | 0,3 % |
| Ethanol (solvant) | 15 % |
| Antioxydant | 0,05 % |
| Eau | qsp 100 % |

Le gel obtenu agit sur les rides. Il peut être appliqué quotidiennement matin et soir pendant un mois.

| **EXEMPLE 3 : Crème de soin du visage (émulsion huile-dans-eau)** | |
|---|---|
| Flunitrazépam | 0,1 % |
| Stéarate de glycérol (émulsionnant) | 2 % |
| Polysorbate 60 (Tween 60 vendu par la société ICI) (émulsionnant) | 1 % |
| Acide stéarique | 1,4 % |
| Triéthanolamine (neutralisant) | 0,7 % |
| Carbomer (Carbopol 940 vendu par la société Goodrich) | 0,4 % |
| Fraction liquide de beurre de karité | 12 % |
| Perhydrosqualène | 12 % |
| Conservateur | 0,3 % |
| Parfum | 0,5 % |
| Antioxydant | 0,05 % |
| Eau | qsp 100 % |

On obtient une crème blanche, onctueuse, qui agit sur les rides et les ridules, et que l'on peut appliquer quotidiennement.

| **EXEMPLE 4 : Crème de soin du visage (émulsion huile-dans-eau)** | |
|---|---|
| Flunitrazépam | 0,2 % |
| Mono-, distéarate de glycérol | 2 % |
| Alcool cétylique | 1,5 % |
| Mélange alcool cétylstéarylique/alcool cétylstéarylique oxy- | |
| éthyléné 33 OE | 7 % |
| Diméthylpolysiloxane | 1,5 % |
| Huile de vaseline | 17,5 % |
| Conservateur | 0,3 % |
| Parfum | 0,5 % |
| Glycérine | 12,5 % |
| Eau | qsp 100 % |

## Revendications

1. Utilisation d'au moins une substance agoniste d'au moins un récepteur associé à au moins un canal chlore présent dans le tissu cutané, sauf la glycine, le glycinate de zinc associé au palmitate de vitamine A dans un rapport 1:1 et l'acide gamma-aminobutyrique, dans une composition cosmétique topique ou pour la fabrication d'une composition dermatologique topique, la composition étant destinée à relaxer et/ou relâcher le tissu cutané.

2. Utilisation d'au moins une substance agoniste d'au moins un récepteur associé à au moins un canal chlore d'au moins une afférence nerveuse cutanée, sauf la glycine, le glycinate de zinc associé au palmitate de vitamine A dans un rapport 1:1 et l'acide gamma-aminobutyrique, dans une composition cosmétique topique ou pour la fabrication d'une composition dermatologique topique, la composition étant destinée à relaxer et/ou relâcher le tissu cutané.

3. Utilisation selon l'une quelconque des revendications 1 ou 2, **caractérisée en ce que** la substance agoniste est choisie dans le groupe comprenant la sérine, la taurine, la β-alanine, la N-(benzyloxycarbonyl)-glycine, l'isoguvacine, l'acide isonipécotique, la 4,5,6,7-tétrahydroisoxazolo-(5,4-c)pyridin-3(2H)-one, les benzodiazépines, les stéroïdes et les barbituriques.

4. Utilisation d'au moins une substance agoniste d'au moins un récepteur associé à au moins un canal chlore présent dans le tissu cutané pour la fabrication d'une composition dermatologique injectable « intralésionnelle » ou « intraridaire », destinée à relaxer et/ou relâcher le tissu cutané.

5. Utilisation d'au moins une substance agoniste d'au moins un récepteur associé à au moins un canal chlore d'au moins une afférence nerveuse cutanée pour la fabrication d'une composition dermatologique injectable « intralésionnelle » ou « intraridaire », destinée à relaxer et/ou relâcher le tissu cutané.

6. Utilisation selon l'une des revendications 4 ou 5, **caractérisée en ce que** la substance agoniste est choisie dans le groupe comprenant la glycine, la sérine, la taurine, la β-alanine, la N-(benzyloxycarbonyl)-glycine, l'acide gamma-aminobutyrique, l'isoguvacine, l'acide isonipécotique, la 4,5,6,7-tétrahydroisoxazolo-(5,4-c)pyridin-3(2H)-one, les benzodiazépines, les stéroïdes et les barbituriques.

7. Utilisation selon l'une quelconque des revendications 1 à 6, en vue de diminuer les rides et/ou les ridules.

8. Utilisation selon l'une quelconque des revendications 1 à 7, **caractérisée en ce que** la substance agoniste est présente dans la composition cosmétique ou dermatologique en une quantité allant de 0,00001 à 20 % en poids par rapport au poids total de la composition.

9. Utilisation selon l'une quelconque des revendications 1 à 8, **caractérisée en ce que** la substance agoniste est présente dans la composition cosmétique ou dermatologique en une quantité allant de 0,01 à 10 % en poids par rapport au poids total de la composition.

10. Utilisation selon l'une quelconque des revendications 1 à 9, **caractérisée en ce que** la composition contient en outre un hydroxyacide et/ou un rétinoïde.

11. Utilisation selon la revendication 10, **caractérisée en ce que** l'hydroxyacide est choisi parmi les α-hydroxyacides ou les β-hydroxyacides, qui peuvent être linéaires, ramifiés ou cycliques, saturés ou insaturés.

12. Utilisation selon la revendication 10, **caractérisée en ce que** le rétinoïde est choisi dans le groupe comprenant l'acide rétinoïque et ses dérivés, le rétinol et ses esters.

## Claims

1. Use of at least one agonist substance of at least one receptor associated with at least one chlorine channel present in cutaneous tissue, except for glycine, zinc glycinate in combination with vitamin A palmitate in a 1:1 ratio and gamma-aminobutyric acid, in a topical cosmetic composition or for the manufacture of a topical dermatological composition, the composition being intended to relax and/or slacken cutaneous tissue.

2. Use of at least one agonist substance of at least one receptor associated with at least one chlorine channel of at least one cutaneous afferent nerve pathway, except for glycine, zinc glycinate in combination with vitamin A palmitate in a 1:1 ratio and gamma-aminobutyric acid, in a topical cosmetic composition or for the manufacture of a topical dermatological composition, the composition being intended to relax and/or slacken cutaneous tissue.

3. Use according to either one of Claims 1 and 2, **characterized in that** the agonist substance is chosen from the group consisting of serine, taurine, β-alanine, N-(benzyloxycarbonyl)glycine, isoguvacine, isonipecotic acid, 4,5,6,7-tetrahydroisoxazolo[5,4-c]pyridin-3(2H)-one, benzodiazepines, steroids and barbiturates.

4. Use of at least one agonist substance of at least one receptor associated with at least one chlorine channel present in cutaneous tissue for the manufacture of an "intralesional" or "intrawrinkle" injectable dermatological composition intended to relax and/or slacken cutaneous tissue.

5. Use of at least one agonist substance of at least one receptor associated with at least one chlorine channel of at least one cutaneous afferent nerve pathway for the manufacture of an "intralesional" or "intrawrinkle" injectable dermatological composition intended to relax and/or slacken cutaneous tissue.

6. Use according to either of Claims 4 and 5, **characterized in that** the agonist substance is chosen from the group consisting of glycine, serine, taurine, β-alanine, N-(benzyloxycarbonyl)glycine, gamma-aminobutyric acid, isoguvacine, isonipecotic acid, 4,5,6,7-tetrahydroisoxazolo(5,4-c)pyrid-3(2H)-one, benzodiazepines, steroids and barbiturates.

7. Use according to any one of Claims 1 to 6, for the purpose of lessening wrinkles and/or fine lines.

8. Use according to any one of Claims 1 to 7, **characterized in that** the agonist substance is present in the cosmetic or dermatological composition in an amount ranging from 0.00001 to 20% by weight with respect to the total weight of the composition.

9. Use according to any one of Claims 1 to 8, **characterized in that** the agonist substance is present in the cosmetic or dermatological composition in an amount ranging from 0.01 to 10% by weight with respect to the total weight of the composition.

10. Use according to any one of Claims 1 to 9, **characterized in that** the composition additionally comprises a hydroxy acid and/or a retinoid.

11. Use according to Claim 10, **characterized in that** the hydroxy acid is chosen from α-hydroxy acids or β-hydroxy acids, which can be linear, branched or cyclic and saturated or unsaturated.

12. Use according to Claim 10, **characterized in that** the retinoid is chosen from the group consisting of retinoic acid and its derivatives and retinol and its esters.

## Patentansprüche

1. Verwendung mindestens eines Agonisten mindestens eines Rezeptors, der mit mindestens einem im Hautgewebe vorliegenden Chlorkanal assoziiert ist, wobei Glycin, Zinkglycinat in Kombination mit Vitamin A-palmitat in einem Verhältnis von 1:1 und gamma-Aminobuttersäure ausgenommen sind, in einer kosmetischen Zusammensetzung zur topischen Anwendung oder für die Herstellung einer dermatologischen Zusammensetzung zur topischen Anwendung, wobei die Zusammensetzung zur Relaxation und/oder Entspannung des Hautgewebes vorgesehen ist.

2. Verwendung mindestens eines Agonisten mindestens eines Rezeptors, der mit mindestens einem Chlorkanal mindestens einer afferenten Nervenfaser der Haut assoziiert ist, wobei Glycin, Zinkglycinat in Kombination mit Vitamin A-palmitat in einem Verhältnis von 1:1 und gamma-Aminobuttersäure ausgenommen sind, in einer kosmetischen Zusammensetzung zur topischen Anwendung oder für die Herstellung einer dermatologischen Zusammensetzung zur topischen Anwendung, wobei die Zusammensetzung zur Relaxation und/oder Entspannung des Hautgewebes vorgesehen ist.

3. Verwendung nach einem der Ansprüche 1 oder 2, **dadurch gekennzeichnet, daß** der Agonist unter Serin, Taurin, β-Alanin, N-(Benzyloxycarbonyl)-glycin, Isoguvacin, Isonipecotsäure, 4,5,6,7-Tetrahydroisoxazolo-(5,4-c)-pyridin-3(2H)-on, Benzodiazepinen, Steroiden und Barbitursäurederivaten ausgewählt ist.

4. Verwendung mindestens eines Agonisten mindestens eines Rezeptors, der mit mindestens einem im Hautgewebe vorliegenden Chlorkanal assoziiert ist, für die Herstellung einer dermatologischen Zusammensetzung, die «in die Läsion hinein» oder «in die Falte hinein» injiziert werden kann und die zur Relaxation und/oder Entspannung des Hautgewebes vorgesehen ist.

5. Verwendung mindestens eines Agonisten mindestens eines Rezeptors, der mit mindestens einem Chlorkanal mindestens einer afferenten Nervenfaser der Haut assoziiert ist, für die Herstellung einer dermatologischen Zusammensetzung, die «in die Läsion hinein» oder «in die Falte hinein» injiziert werden kann und die zur Relaxation und/oder Entspannung des Hautgewebes vorgesehen ist.

6. Verwendung nach einem der Ansprüche 4 oder 5, **dadurch gekennzeichnet, daß** der Agonist unter Glycin, Serin, Taurin, β-Alanin, N-(Benzyloxycarbonyl)-glycin, gamma-Aminobuttersäure, Isoguvacin, Isonipecotsäure, 4,5,6,7-Tetrahydroisoxazolo-(5,4-c)-pyridin-3(2H)-on, Benzodiazepinen, Steroiden und Barbitursäurederivaten ausgewählt ist.

7. Verwendung nach einem der Ansprüche 1 bis 6 für die Abschwächung von Falten und/oder Fältchen.

8. Verwendung nach einem der Ansprüche 1 bis 7, **dadurch gekennzeichnet, daß** der Agonist in der kosmetischen oder dermatologischen Zusammensetzung in einer Menge von 0,00001 bis 20 Gew.- %, bezogen auf das Gesamtgewicht der Zusammensetzung, vorliegt.

9. Verwendung nach einem der Ansprüche 1 bis 8, **dadurch gekennzeichnet, daß** der Agonist in der kosmetischen oder dermatologischen Zusammensetzung in einer Menge von 0,01 bis 10 Gew.-%, bezogen auf das Gesamtgewicht der Zusammensetzung, vorliegt.

10. Verwendung nach einem der Ansprüche 1 bis 9, **dadurch gekennzeichnet, daß** die Zusammensetzung außerdem eine Hydroxysäure und/oder ein Retinoid enthält.

11. Verwendung nach Anspruch 10, **dadurch gekennzeichnet, daß** die Hydroxysäure unter den α-Hydroxysäuren oder β-Hydroxysäuren ausgewählt ist, die geradkettig, verzweigt oder cyclisch, gesättigt oder ungesättigt vorliegen können.

12. Verwendung nach Anspruch 10, **dadurch gekennzeichnet, daß** das Retinoid unter Retinsäure und ihren Derivaten und Retinol und seinen Estern ausgewählt ist.
